(19) Europäisches Patentamt
European Patent Office
Office européen des brevets

(11) **EP 4 582 073 A1**

(12) **EUROPEAN PATENT APPLICATION**
published in accordance with Art. 153(4) EPC

(43) Date of publication:
09.07.2025 Bulletin 2025/28

(21) Application number: 23860554.7

(22) Date of filing: 01.09.2023

(51) International Patent Classification (IPC):
*A61K 6/15* (2020.01)     *A61K 6/20* (2020.01)
*A61K 6/60* (2020.01)     *A61K 6/77* (2020.01)
*A61K 6/836* (2020.01)

(52) Cooperative Patent Classification (CPC):
A61K 6/15; A61K 6/20; A61K 6/60; A61K 6/77;
A61K 6/836

(86) International application number:
PCT/JP2023/032152

(87) International publication number:
WO 2024/048794 (07.03.2024 Gazette 2024/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 02.09.2022 JP 2022140300

(71) Applicant: Kuraray Noritake Dental Inc.
Kurashiki-shi, Okayama 710-0801 (JP)

(72) Inventors:
• EMOTO, Tomohiro
  Miyoshi-shi, Aichi 470-0293 (JP)
• KASHIKI, Nobusuke
  Miyoshi-shi, Aichi 470-0293 (JP)
• TAKEUCHI, Yuta
  Miyoshi-shi, Aichi 470-0293 (JP)

(74) Representative: D Young & Co LLP
3 Noble Street
London EC2V 7BQ (GB)

(54) **DENTAL PORCELAIN PASTE, DENTAL PORCELAIN KIT, AND METHOD FOR PRODUCING DENTAL PORCELAIN PROSTHESIS**

(57)     The present invention relates to a dental porcelain paste comprising glass powder (A), an organic solvent (B), and a coloring agent (C) that is decolorable by baking, and satisfying at least one condition selected from a condition (1): a color difference $\Delta E^*$ calculated from a color tone obtained by colorimetrically measuring in the $L^*a^*b^*$ color space the dental porcelain paste having a thickness of 0.5 mm before baking and a color tone obtained by colorimetrically measuring in the $L^*a^*b^*$ color space a baked product having a thickness of 0.5 mm obtained by baking the dental porcelain paste in vacuum at 740°C is 8.5 or more, and a condition (2): a brightness difference $\Delta L^*$ calculated from a brightness obtained by colorimetrically measuring in the $L^*a^*b^*$ color space the dental porcelain paste having a thickness of 0.5 mm before baking and a brightness obtained by colorimetrically measuring in the $L^*a^*b^*$ color space a baked product having a thickness of 0.5 mm obtained by baking the dental porcelain paste in vacuum at 740°C is 6.5 or more; a dental porcelain kit; and a method for producing a dental prosthesis.

EP 4 582 073 A1

## Description

Technical Field

The present invention relates to a dental porcelain paste, a dental porcelain kit including the dental porcelain paste, and a method for producing a dental porcelain prosthesis using the dental porcelain paste.

Background Art

Ceramics have transparency and color tone similar to those of natural teeth, and therefore are being an indispensable material for producing dental crowns, which require aesthetic quality.

In general, a dental crown is produced by repeating an operation of building up a dental admixture containing a porcelain material, one or more kind selected from water and an organic solvent, and the like, or a porcelain material in the form of paste formed by mixing one or more kind selected from water and an organic solvent, and the like with ceramics in advance, on a frame (such as a metal frame or a ceramic frame) covering the abutment tooth, and then baking.

A porcelain material in the form of powder requires a complicated procedure of mixing with a solvent every time performing the operation, and requires a skilled operation for regulating the mixing ratio corresponding to the desired color tone, and the viscosity thereof, but the porcelain material in the form of paste contains a porcelain material and a solvent that have been mixed to have a viscosity enabling the operation by the dental technician with good handleability, and therefore can reduce the period of time of dental technician work.

For example, PTL 1 describes a paste-like dental porcelain material containing a coloring agent (A) decolorable by baking, an organic solvent (B), and porcelain powder (C), in which the porcelain powder (C) contains an inorganic pigment.

Examples of the base material used in a dental prosthesis of a ceramic material include zirconium oxide (zirconia), aluminum oxide (alumina), feldspar glass, disilicate glass. In recent years, there are also increasing opportunities for these materials to be processed using a dental CAD/CAM system and used as a dental prosthesis.

Known representative examples of the dental prosthesis of a ceramic material include dental prostheses of a porcelain fused to metal type (abbr.: PFM), a porcelain fused to zirconia type (abbr.: PFZ), a full contour zirconia type (abbr.: FCZ), and a lithium disilicate type (abbr.: LDS).

For finishing the dental prostheses, a dental porcelain material, such as a stain porcelain material and a glaze porcelain material, has been used.

For example, PTL 2 describes a color tone regulating composition for a ceramic based dental crown porcelain material containing two or more kinds of aluminosilicate glass and a coloring agent, in which the two or more kinds of aluminosilicate glass each satisfy the particular requirement.

Citation List

Patent Literatures

PTL 1: JP 2017-193492 A
PTL 2: JP 2009-207743 A

Summary of Invention

Technical Problem

In general, a glaze porcelain material is used mainly for the purpose of glazing a dental prosthesis, and is demanded to have a small color difference from the base material (which may also be hereinafter referred to as a "substrate") after baking. Therefore, the porcelain paste used as the glaze porcelain material is transparent to translucent and has substantially no color saturation before baking. However, with the use of the porcelain paste of this type, it is difficult to distinguish between the portion having the glaze porcelain material coated thereon and the portion having no glaze porcelain material coated thereon, and to confirm the coating thickness, and as a result, uncoated areas of the porcelain material and coating unevenness thereof tend to occur. The uncoated areas of the porcelain material and the coating unevenness thereof cause problems including the deteriorated aesthetic quality, the prolonged period of time required for adjusting the contacts of the opposing surface or the adjacent surface, the increased numbers of coating and baking operations for compensating the uncoated areas and the insufficient coating areas of the porcelain material, and the like.

Therefore, for enabling the use of the dental porcelain paste as the glaze porcelain material, it is demanded that in coating the dental porcelain paste on the substrate before baking, the capability of visually distinguishing the portion having the dental porcelain paste coated thereon (which may be hereinafter referred simply to as "distinguishability") is further enhanced.

PTL 1 makes a mention of the color difference of the paste before and after baking. However, the mention focuses on the problem that in the case where the coloration is performed with a stain porcelain material, the color tone of the porcelain material is changed before and after baking, and therefore a skilled technique is required for adjusting the color tone of the porcelain material in building up through estimation of the color tone after baking, and the mention is made on the suppression of the color difference of the paste-like dental porcelain material before and after baking. Accordingly, the paste-like dental porcelain materials described in the examples of PTL 1 are examples relating mainly to the stain porcelain material, and a glaze porcelain material is not described therein. In the case where the paste-like dental porcelain material described in PTL 1 is used as a glaze porcelain material, a further improvement is required in the distinguishability described above. Furthermore, as described above, the glaze porcelain material is used for the purpose mainly of glazing, and therefore the color difference between the baked product obtained by baking the dental porcelain paste and the substrate is desirably small.

PTL 2 does not make a mention on the problem in the use of the glaze porcelain material described above.

Consequently, in the case where a dental porcelain paste is made to be applicable particularly to the glaze porcelain material, it is demanded that the distinguishability is further enhanced.

Under the circumstances, an object of the present invention is to provide a dental porcelain paste that is excellent in distinguishability of the dental porcelain paste in a coating operation before baking.

Solution to Problem

As a result of the earnest investigations by the present inventors, it has been found that the problem can be solved by the use of a dental porcelain paste that contains glass powder (A), an organic solvent (B), and a coloring agent (C) decolorable by baking, and satisfies a particular condition, and thus the present invention has been completed.

Specifically, the present invention encompasses the following inventions.

[1] A dental porcelain paste comprising glass powder (A), an organic solvent (B), and a coloring agent (C) decolorable by baking, and satisfying at least one condition selected from the following conditions (1) and (2):

condition (1): a color difference $\Delta E^*$ calculated from a color tone obtained by colorimetrically measuring in the $L^*a^*b^*$ color space the dental porcelain paste having a thickness of 0.5 mm before baking and a color tone obtained by colorimetrically measuring in the $L^*a^*b^*$ color space a baked product having a thickness of 0.5 mm obtained by baking the dental porcelain paste in vacuum at 740°C is 8.5 or more, and
condition (2): a brightness difference $\Delta L^*$ calculated from a brightness obtained by colorimetrically measuring in the $L^*a^*b^*$ color space the dental porcelain paste having a thickness of 0.5 mm before baking and a brightness obtained by colorimetrically measuring in the $L^*a^*b^*$ color space a baked product having a thickness of 0.5 mm obtained by baking the dental porcelain paste in vacuum at 740°C is 6.5 or more.

[2] The dental porcelain paste according to the item [1], in which a color difference $\Delta E1^*$ calculated from a color tone obtained by colorimetrically measuring in the $L^*a^*b^*$ color space a base material and a color tone obtained by colorimetrically measuring in the $L^*a^*b^*$ color space the dental porcelain paste coated on the base material to a thickness of 0.5 mm is 11.0 or more.

[3] The dental porcelain paste according to the item [1] or [2], in which a color difference $\Delta E2^*$ calculated from a color tone obtained by colorimetrically measuring in the $L^*a^*b^*$ color space a base material and a color tone obtained by colorimetrically measuring in the $L^*a^*b^*$ color space a baked product obtained by coating the dental porcelain paste on the base material to make a thickness after baking of 65 $\mu$m, and baking in vacuum at 740°C is 3.0 or less.

[4] The dental porcelain paste according to any one of the items [1] to [3], in which the dental porcelain paste has a content of the component (C) of 0.001 part by mass or more and less than 0.60 part by mass with respect to 100 parts by mass in total of the components (A) and (B).

[5] The dental porcelain paste according to any one of the items [1] to [4], in which the component (C) contains an organic colorant that is dissolvable in the component (B).

[6] The dental porcelain paste according to the item [5], in which the organic colorant is an aromatic based organic colorant.

[7] The dental porcelain paste according to the item [5] or [6], in which the organic colorant is at least one kind selected from the group consisting of an anthraquinone based compound, an azo based compound, a xanthene based compound, a porphyrin based compound, a phthalocyanine based compound, and a triarylmethane base compound.

[8] The dental porcelain paste according to any one of the items [1] to [7], which comprises a fluorescent agent.

[9] The dental porcelain paste according to the item [8], in which the dental porcelain paste has a content of the fluorescent agent of 0.001 to 0.50 part by mass with respect to 100 parts by mass in total of the components (A) and (B).

[10] The dental porcelain paste according to any one of the items [1] to [9], which comprises an inorganic pigment.

[11] A dental porcelain kit comprising the dental porcelain paste according to any one of the items [1] to [10] and a color reference for being the reference for a coating thickness of the dental porcelain paste.

[12] A method for producing a dental prosthesis, comprising a step (I) of coating the dental porcelain paste according to any one of the items [1] to [10] on a base material, and a step (II) of baking the dental porcelain paste coated on the base material.

[13] The method for producing a dental prosthesis according to the item [12], in which a baking temperature in the step (II) is 700°C or more.

[14] The method for producing a dental prosthesis according to the item [12] or [13], in which the step (I) includes using a color reference for being the reference for a coating thickness of the dental porcelain paste.

Advantageous Effects of Invention

The present invention can provide a dental porcelain paste that is excellent in distinguishability of the dental porcelain paste in a coating operation before baking.

Description of Embodiments

The present invention will be described in detail with reference to embodiments below.

In the description herein, the upper limit values and the lower limit values of the numeral range (such as the content of the component, the value calculated from the components, and the property value) can be appropriately combined.

Therefore, in the description herein, the lower limit values and the upper limit values described in a stepwise manner for the numeral range can be independently combined. For example, based on the description "preferably 10 to 90, and more preferably 30 to 60" for the same item, the "preferred lower limit value (10)" and the "more preferred upper limit value (60)" can be combined to make a range of "10 to 60".

As for the numeral ranges, for example, based on the description "preferably 10 to 90, and more preferably 30 to 60", only the lower limit value can be defined as "10 or more" or "30 or more" without particularly limiting the upper limit value, and similarly, only the upper limit value can be defined as "90 or less" or "60 or less" without particularly limiting the lower limit value.

Unless otherwise indicated, the description "10 to 90" simply for the numeral range means a range of 10 or more and 90 or less.

As similar to the above, for example, based on the description "preferably 10 or more, and more preferably 30 or more" and the description "preferably 90 or less, and more preferably 60 or less" for the same item, the "preferred lower limit value (10)" and the "more preferred upper limit value (60)" can be combined to make a range of "10 or more and 60 or less". As similar to the above, only the lower limit value can be defined as "10 or more" or "30 or more", and similarly, only the upper limit value can be defined as "90 or less" or "60 or less". The same is also applied to the case where the upper limit of the numeral range is "less than" and the case where the lower limit thereof is "more than".

In the description herein, unless otherwise indicated, as described above, the term "distinguishability" means the capability of visually distinguishing between the portion having the dental porcelain paste before baking coated thereon and the portion having no dental porcelain paste coated thereon, when the dental porcelain paste is coated on the substrate. Specifically it means the capability that is evaluated by the method described in the examples.

In the description herein, unless otherwise indicated, in the case where the term "baked product" is simply described, the "baked product" means a baked product that is obtained by baking the dental porcelain paste.

[Dental Porcelain Paste]

The dental porcelain paste as one embodiment of the present invention (which may be hereinafter referred simply to as a "porcelain paste") contains glass powder (A) (which may be hereinafter referred simply to as a "component (A)"), an organic solvent (B) (which may be hereinafter referred simply to as a "component (B)"), and a coloring agent (C) decolorable by baking (which may be hereinafter referred simply to as a "component (C)"), and satisfies at least one condition selected from the following conditions (1) and (2).

Condition (1): A color difference $\Delta E^*$ calculated from a color tone obtained by colorimetrically measuring in the L*a*b* color space the dental porcelain paste having a thickness of 0.5 mm before baking and a color tone obtained by colorimetrically measuring in the L*a*b* color space a baked product having a thickness of 0.5 mm obtained by baking the dental porcelain paste in vacuum at 740°C is 8.5 or more.

Condition (2): A brightness difference $\Delta L^*$ calculated from a brightness obtained by colorimetrically measuring in the L*a*b* color space the dental porcelain paste having a thickness of 0.5 mm before baking and a brightness obtained by colorimetrically measuring in the L*a*b* color space a baked product having a thickness of 0.5 mm obtained by baking the dental porcelain paste in vacuum at 740°C is 6.5 or more.

The dental porcelain paste can satisfy at least one condition selected from the conditions (1) and (2) by containing the coloring agent (C) decolorable by baking.

<Condition (1)>

In the case where the color difference $\Delta E^*$ relating to the condition (1) is 8.5 or more, the difference between the color tone of the porcelain paste before baking and the color tone of the baked product obtained by baking the porcelain paste is large. Accordingly, for example, in the case where the color tone of the substrate on which the baked product is to be formed and the color tone of the baked product are close to each other, in coating the porcelain paste before baking on the substrate, the value of $\Delta E^*$ or 8.5 or more allows the portion having the porcelain paste coated thereon to be clearly distinguished easily, resulting in the enhanced distinguishability of the porcelain paste.

The color difference $\Delta E^*$ is preferably 11.0 or more, more preferably 15.0 or more, further preferably 20.0 or more, still further preferably 30.0 or more, and still more further preferably 40.0 or more, from the standpoint of enhancing the distinguishability of the porcelain paste.

The upper limit of the color difference $\Delta E^*$ is not particularly limited, and for example, the color difference $\Delta E^*$ is preferably 95.0 or less, more preferably 90.0 or less, further preferably 85.0 or less, and still further preferably 80.0 or less.

As described above, the lower limit values and the upper limit values described in a stepwise manner each can be independently combined. For example, in one embodiment of the present invention, the color difference $\Delta E^*$ may be 8.5 to 95.0, and is preferably 11.0 to 95.0, more preferably 15.0 to 90.0, further preferably 20.0 to 85.0, still further preferably 30.0 to 80.0, and still more further preferably 40.0 to 80.0.

The color difference $\Delta E^*$ relating to the condition (1) can be specifically calculated by the method described in the examples.

<Condition (2)>

In the case where the brightness difference $\Delta L^*$ relating to the condition (2) is 6.5 or more, in coating the porcelain paste before baking on the substrate, the porcelain paste coated thereon is clearly distinguished easily due to the same mechanism as in the description relating to the condition (1) above, resulting in the enhanced distinguishability of the porcelain paste.

The brightness difference $\Delta L^*$ is preferably 11.0 or more, more preferably 15.0 or more, further preferably 18.0 or more, still further preferably 20.0 or more, and still more further preferably 25.0 or more, from the standpoint of enhancing the distinguishability of the porcelain paste.

The upper limit of the brightness difference $\Delta L^*$ is not particularly limited, and for example, the color difference $\Delta L^*$ is preferably 100.0 or less, more preferably 80.0 or less, and further preferably 60.0 or less.

As described above, the lower limit values and the upper limit values described in a stepwise manner each can be independently combined. For example, in one embodiment of the present invention, the brightness difference $\Delta L^*$ may be 6.5 to 100.0, and is preferably 11.0 to 100.0, more preferably 15.0 to 80.0, further preferably 18.0 to 80.0, still further preferably 20.0 to 60.0, and still more further preferably 25.0 to 60.0.

The color difference $\Delta L^*$ relating to the condition (2) can be specifically calculated by the method described in the examples.

In the dental porcelain paste, the color difference $\Delta E1^*$ calculated from the color tone obtained by colorimetrically measuring in the L*a*b* color space the base material and the color tone obtained by colorimetrically measuring in the L*a*b* color space the dental porcelain paste coated on the base material to a thickness of 0.5 mm is preferably 11.0 or more, more preferably 15.0 or more, further preferably 20.0 or more, still further preferably 30.0 or more, and still more further preferably 40.0 or more, from the standpoint of further enhancing the distinguishability of the dental porcelain paste.

The upper limit value of the color difference $\Delta E1^*$ is not particularly limited, and for example, the color difference $\Delta E1^*$ is preferably 95.0 or less, more preferably 90.0 or less, further preferably 85.0 or less, and still further preferably 80.0 or less.

As described above, the lower limit values and the upper limit values described in a stepwise manner each can be independently combined. For example, in one embodiment of the present invention, the color difference $\Delta E1^*$ is preferably 11.0 to 95.0, more preferably 15.0 to 90.0, further preferably 20.0 to 85.0, still further preferably 30.0 to 80.0, and still more further preferably 40.0 to 80.0.

The color difference ΔE1* can be specifically calculated by the method described in the examples. In the description herein, the "base material" used in calculating the color difference ΔE1* means a base material that is used for calculating the color difference ΔE1*, and specifically means the particular base material described in the examples described later. Therefore, the kind of the "base material" that is a target on which the porcelain paste is to be coated is not limited thereby, and similarly the kind of the "base material" that is a target on which the porcelain paste is to be coated in the step (I) described later is also not limited thereby.

In the dental porcelain paste, for example, the color difference ΔE2* calculated from the color tone obtained by colorimetrically measuring in the L*a*b* color space the base material and the color tone obtained by colorimetrically measuring in the L*a*b* color space the baked product obtained by coating the dental porcelain paste on the base material to make a thickness after baking of 65 μm, and baking in vacuum at 740°C is preferably 3.0 or less, more preferably 2.5 or less, further preferably 2.0 or less, still further preferably 1.5 or less, and still more further preferably 1.2 or less, from the standpoint of reducing the influence of the baked product on the color tone of the substrate.

The lower limit of the color difference ΔE2* is not particularly limited, and for example, the color difference ΔE2* is preferably 0.0 or more.

As described above, the lower limit values and the upper limit values described in a stepwise manner each can be independently combined. For example, in one embodiment of the present invention, the color difference ΔE2* is preferably 0.0 to 3.0, more preferably 0.0 to 2.5, further preferably 0.0 to 2.0, still further preferably 0.0 to 1.5, and still more further preferably 0.0 to 1.2.

The color difference ΔE2* can be specifically calculated by the method described in the examples. In the description herein, the "base material" used in calculating the color difference ΔE2* means the base material described in the examples described later.

The components contained in the dental porcelain paste as one embodiment of the present invention and the like are described below in order.

<Glass Powder (A)>

The glass powder (A) is not particularly limited, preferably a material capable of being applied to the dental use, and may contain crystals.

Examples of the material of the component (A) include glass containing $SiO_2$ as a major component (i.e., a component having the largest content in the glass) or crystallized glass. The glass may contain $Al_2O_3$, $B_2O_3$, $ZnO$, $K_2O$, $Na_2O$, $Li_2O$, $ZrO_2$, $CaO$, $MgO$, $Sb_2O_3$, $CeO_2$, $BaO$, $SnO_2$, and the like in addition to $SiO_2$, and for example, at least one kind selected from the group consisting of amorphous type potassium aluminosilicate glass ($4SiO_2 \cdot Al_2O_3 \cdot K_2O$), leucite crystal type potassium aluminosilicate glass, fluorapatite glass and lithium silicate glass can be preferably used. Among these, amorphous type potassium aluminosilicate glass is more preferred. Examples of the crystal include leucite, potassium feldspar, fluorphlogopite, diopside, mica, β-spodumene ($LiAlSi_2O_6$), calcium β-metaphosphate, apatite, magnesium titanate, β- eucryptite, and alumina.

One kind of the component (A) may be used alone, or two or more kinds thereof may be appropriately used in combination, depending on the purpose of the porcelain paste.

The raw material substance of the glass to be the material of the component (A) may be ceramic materials having been ordinarily widely used, and examples thereof used include an oxide, such as $SiO_2$, $Al_2O_3$, $B_2O_3$, $ZnO$, $K_2O$, $Na_2O$, $Li_2O$, $ZrO_2$, $CaO$, $MgO$, $Sb_2O_3$, $CeO_2$, $BaO$, and $SnO_2$; a substance capable of becoming the oxide, such as $SiO_2$, $Al_2O_3$, $B_2O_3$, $ZnO$, $K_2O$, $Na_2O$, $Li_2O$, $ZrO_2$, $CaO$, $MgO$, $Sb_2O_3$, $CeO_2$, $BaO$, and $SnO_2$, in heating in the air; and a mixture of the oxide and the substance capable of becoming the oxide. In this case, the glass composition to be obtained is calculated in advance, and the formulation is determined thereby, according to which the raw material substances are mixed. The method of mixing the raw material substances is not particularly limited, and the raw material substances are preferably dispersed uniformly.

The mixed raw material substances are heated to approximately 700°C or more. The method of heating is not particularly limited, and it suffices that the raw material substances are completely melted to form a uniform molten matter. The method of cooling the molten matter is also not particularly limited, and may be air cooling or the like.

The glass gob obtained in this manner is pulverized and classified to provide the component (A) having a regulated particle size. The method of pulverizing and classifying the glass gob is not particularly limited, and examples of the pulverizer include a compression pulverizer, such as a jaw crusher and a cone crusher; a ball mill, such as a vibration ball mill and a planetary ball mill; a medium agitation type pulverizer, such as a tower pulverizer, an agitation tank pulverizer, and an annular type pulverizer; and a high-speed rotation impact pulverizer, such as a pin mill and a disk mill; and also include a roll mill, a jet pulverizer, and an autogenous pulverizer. Examples of the classifier include a sieve classifier, such as a vibration sieve and a shifter; a centrifugal classifier, such as a cyclone; and a wet classifier, such as a sedimentation classifier. The pulverizer and the classifier each are preferably an apparatus coated with a resin or glass for avoiding contamination of metal impurities.

The average particle diameter of the component (A) is preferably 1 to 30 $\mu$m, more preferably 2 to 20 $\mu$m, and further preferably 3 to 9 $\mu$m. The average particle diameter that is 1 $\mu$m or more is preferred since the decrease in transparency of the prosthesis can be easily suppressed. The average particle diameter that is 30 $\mu$m or less is preferred from the standpoint of facilitating the coating operation of the porcelain paste.

The average particle diameter of the component (A) means the volume based average particle diameter (D50) that can be obtained through measurement using the laser diffraction scattering method, and can be measured, for example, by the method described in the examples.

The glass transition temperature of the component (A) is preferably 400 to 600°C, more preferably 420 to 580°C, and further preferably 450 to 550°C, from the standpoint of enabling the baking at a lower temperature and shortening the baking time. The softening point of the component (A) is preferably 500 to 680°C, more preferably 520 to 650°C, and further preferably 550 to 630°C, from the same standpoint. The glass transition temperature that is 400°C or more or the softening point that is 500°C or more is preferred since the component (A) can be prevented from sagging in baking. On the other hand, the glass transition temperature that is 600°C or less or the softening point that is 680°C or less is preferred since the baking can be performed at a low temperature, and thereby a ceramic frame formed of lithium disilicate glass ceramics or the like can be prevented from being deformed in baking at a high temperature.

The linear thermal expansion coefficient (50 to 500°C) of the component (A) may be appropriately selected depending on the material of the substrate on which the porcelain paste is to be coated, and is not particularly limited, and may be $4.0 \times 10^{-6}$ to $6.0 \times 10^{-6}$ /°C, for example, may be $6.1 \times 10^{-6}$ to $13.5 \times 10^{-6}$ /°C, and may be $6.3 \times 10^{-6}$ to $12.5 \times 10^{-6}$ /°C. For example, in the case where the porcelain paste is applied to the substrate formed of zirconia as a major component, the linear thermal expansion coefficient of the component (A) is preferably $9.0 \times 10^{-6}$ to $11.0 \times 10^{-6}$ /°C. For example, in the case where the porcelain paste is applied to the substrate formed of alumina as a major component, the linear thermal expansion coefficient of the component (A) is preferably $6.1 \times 10^{-6}$ to $8.8 \times 10^{-6}$ /°C. The linear thermal expansion coefficient can be measured, for example, by heating a specimen from room temperature (25°C) to 500°C with a thermal analyzer, TMA 120 (available from Seiko Instruments Inc., temperature rise rate: 5°C/min). The linear thermal expansion coefficient can be regulated by the known method, and for example, can be regulated by the content of $K_2O$.

The content of the component (A) in the dental porcelain paste is preferably 40 to 80 parts by mass, more preferably 45 to 75 parts by mass, and further preferably 50 to 70 parts by mass, with respect to 100 parts by mass in total of the component (A) and the component (B).

<Organic Solvent (B)>

Examples of the organic solvent (B) include

an ester, such as dimethyl phthalate, diethyl phthalate, and dibutyl phthalate;
a monohydric alcohol, such as methanol, ethanol, 1-propanol, 2-propanol, isopropanol, 1-butanol, 2-butanol, 1-heptanol, 2-heptanol, 3-heptanol, 1-hexanol, 2-hexanol, 3-hexanol, 2-methyl-1-pentanol, 3-methyl-1-pentanol, 4-methyl-1-pentanol, 2-methyl-2-pentanol, 2-methyl-3-pentanol, 2,2-dimethyl-1-butanol, and 2-ethyl-1-butanol;
a polyhydric alcohol, such as 1,2-ethanediol, 1,2-propanediol, 1,3-propanediol, 1,2-butanediol, 1,3-butanediol, 1,4-butanediol, 2,3-butanediol, 1,2-pentanediol, 1,5-pentanediol, 2,4-pentanediol, 1,2-hexanediol, 2,5-hexanediol, 1-methyl-1,3-propanediol, 2-methyl-1,3-propanediol, 2-methyl-1,4-butanediol, 3-methyl-1,3-butanediol (alias: isoprene glycol), 2-methyl-2,4-pentanediol, 3-methyl-1,5-pentanediol, 2,4-diethyl-1,5-pentanediol, 2-ethyl-1,3-hexanediol, 3-(benzyloxy)-1,2-propanediol, 4-(benzyloxy)-1,2-butanediol, ethylene glycol, diethylene glycol, triethylene glycol, tetraethylene glycol, polyethylene glycol (molecular weight: 200 to 600), propylene glycol, dipropylene glycol, polypropylene glycol, glycerol, 1,2,3-butanetriol, and 1,2,6-hexanetriol;
an aromatic alcohol, such as 2-phenoxyethanol and benzyl alcohol;
a polyhydric alcohol monoether, such as ethylene glycol monomethyl ether, ethylene glycol monoethyl ether, propylene glycol monomethyl ether, propylene glycol monoethyl ether, ethylene glycol monobutyl ether, diethylene glycol monomethyl ether, diethylene glycol monoethyl ether, propylene glycol monopropyl ether, dipropylene glycol monoethyl ether, tripropylene glycol monomethyl ether, diethylene glycol monobutyl ether, and triethylene glycol monomethyl ether; and
a (meth)acrylic based polymerizable monomer, such as 2-hydroxyethyl (meth)acrylate.

One kind of the component (B) may be used alone, or two or more kinds thereof may be appropriately used in combination.

The component (B) is preferably at least one kind selected from the group consisting of a polyhydric alcohol, an aromatic alcohol, and a polyhydric alcohol monoether, and more preferably at least one kind selected from the group consisting of a polyhydric alcohol and an aromatic alcohol.

The component (B) is further preferably at least one kind selected from the group consisting of 1,2-propanediol, 1,3-butanediol, 1,4-butanediol, 3-methyl-1,3-butanediol (alias: isoprene glycol), 3-methyl-1,5-pentanediol, 2-ethyl-1,3-hexanediol, and 2-phenoxyethanol.

The component (B) is preferably an alcohol that is in a liquid state at 20°C.

The boiling point of the component (B) is preferably 100 to 300°C, more preferably 100 to 280°C, and further preferably 100 to 250°C.

The content of the component (B) in the dental porcelain paste is not particularly limited, and is preferably 20 to 60 parts by mass, more preferably 25 to 55 parts by mass, and further preferably 30 to 50 parts by mass, with respect to 100 parts by mass in total of the component (A) and the component (B). The content of the component (B) that is 20 parts by mass or more is preferred since the kneadability of the component (A) and the component (B) is enhanced, which are therefore can be easily formed into a paste. The content of the component (B) that is 60 parts by mass or less is preferred since the glass content in the porcelain paste can be easily secured, which facilitates the coating operation of the porcelain paste.

<Coloring Agent (C) decolorable by Baking>

The coloring agent (C) decolorable by baking will be decolored in baking, and thereby in coating the porcelain paste before baking on the substrate, the portion having the porcelain paste coated thereon can be clearly distinguished easily, whereas the coloration of the baked product obtained by baking the porcelain paste due to the component (C) is decolored, decreasing the influence of the baked product on the color tone of the substrate.

In the description herein, the "decolorable by baking" for the component (C) means not only the decoloration of the coloring agent itself in baking, but also the decoloration of the color derived from the component (C) in the porcelain paste in baking. Accordingly, it is considered that the mechanism of the decoloration of the coloration due to the component (C) includes the burnout of the component (C).

As described above, the dental porcelain paste can satisfy at least one condition selected from the conditions (1) and (2) by using the component (C).

In the description herein, the "decoloration" for the component (C) means that in the case where a porcelain paste (I) containing the glass powder (A) and the organic solvent (B) but does not contain the coloring agent (C) decolorable by baking and a porcelain paste (II) containing the porcelain paste (I) having the coloring agent (C) decolorable by baking added thereto are baked at 700°C or more, the color difference $\Delta E^*ab$ between the baked product of the porcelain paste (I) and the baked product of the porcelain paste (II) is preferably less than 0.50, more preferably less than 0.30, and further preferably less than 0.20. The condition in baking except for the baking temperature and the measurement method of the color difference $\Delta E^*ab$ are as described in the examples described later.

The component (C) preferably contains an organic colorant that is dissolvable in the component (B). The expression "dissolvable in the component (B)" means that in the case where 1 g thereof is dissolved in 10 mL of the component (B) at 25°C, no white turbidity is visually observed.

The content of the organic colorant is preferably 60% by mass or more, more preferably 80% by mass or more, further preferably 90% by mass or more, and still further preferably 98% by mass or more, and is 100% by mass or less, based on the component (C) as 100% by mass.

As described above, the lower limit values and the upper limit values described in a stepwise manner each can be independently combined. For example, in one embodiment of the present invention, the content of the organic colorant is preferably 60 to 100% by mass, more preferably 80 to 100% by mass, further preferably 90 to 100% by mass, and still further preferably 98 to 100% by mass, based on the component (C) as 100% by mass.

The organic colorant is preferably an aromatic based organic colorant.

The aromatic based organic colorant is an organic colorant that has one or more aromatic group that may be substituted, and is preferably an organic colorant having an auxochrome group in addition to a chromophore group.

The chromophore group is not particularly limited, as long as being an atomic group that causes coloration through bonding to the aromatic ring. Examples of the atomic group include a nitro group, an azo group, a ketimide group (>C=N-), a carbonyl group, a carbon-carbon double bond, a carbon-carbon triple bond, a carbon-nitrogen multiple bond, a thiocarbonyl group, a nitroso group, and an azoxy group. The aromatic organic colorant may contain one kind of the atomic groups alone, or two or more kinds thereof as an appropriate combination.

Examples of the auxochrome group include a hydroxy group, an amino group, a carboxy group, a sulfone group, and a halogen atom. The aromatic organic colorant may contain one kind of the auxochrome groups alone, or two or more kinds thereof as an appropriate combination.

The organic colorant is preferably at least one kind selected from the group consisting of an anthraquinone based compound, an azo based compound, a xanthene based compound, a porphyrin based compound, a phthalocyanine based compound, and a triarylmethane base compound, more preferably at least one kind selected from the group consisting of an anthraquinone based compound, an azo based compound, a xanthene based compound, and a phthalocyanine based compound, and further preferably at least one kind selected from the group consisting of an azo based compound and a phthalocyanine based compound. These compounds have the good coloring capability, therefore can reduce the content of the component (C) in the porcelain paste and as described above, are preferred from the stand point that the burnout residue of the component (C) in baking can be reduced, suppressing the occurrence of bubbles and carbonization in the resulting baked product.

Specific examples of the organic colorant are not particularly limited, as long as exerting the effects of the present invention, and for example include the following organic colorants and the like. In the following description, for example, the expression "C.I. Direct Blue (25, 86, 90, 108)" means "C.I. Direct Blue 25, C.I. Direct Blue 86, C.I. Direct Blue 90, and C.I. Direct Blue 108".

- · C.I. Direct Blue (25, 86, 90, 108)
- · C.I. Solvent Blue (11, 14, 18, 25, 37, 44, 45, 49, 58, 59, 68, 78, 94)
- · C.I. Acid Blue (1, 3, 7, 9, 15, 83, 90, 103, 104, 158, 161, 249)
- · C.I. Basic Blue (1, 3, 7, 9, 25, 105)
- · C.I. Disperse Blue (198)
- · C.I. Mordant Blue (1)
- · C.I. Solvent Red (25, 27, 30, 35, 49, 83, 89, 100, 122, 138, 149, 150, 160, 179, 218, 230)
- · C.I. Acid Red (6, 8, 9, 13, 14, 18, 26, 27, 51, 52, 87, 88, 89, 92, 94, 97, 111, 114, 115, 134, 145, 151, 154, 180, 183, 184, 186, 198)
- · C.I. Direct Red (20, 37, 39, 44)
- · C.I. Basic Red (12, 13)
- · C.I. Disperse Red (5, 7, 13, 17, 58)
- · C.I. Solvent Black (3, 5, 7, 27, 28, 29, 35, 45, 46)
- · C.I. Solvent Yellow (2, 5, 14, 15, 16, 19, 21, 33, 56, 62, 77, 83, 93, 162)
- · C.I. Disperse Yellow (3, 4, 7, 31, 54, 61, 201)
- · C.I. Direct Yellow (1, 11, 12, 28)
- · C.I. Acid Yellow (1, 3, 11, 17, 23, 38, 40, 42, 76, 98)
- · C.I. Basic Yellow (1)
- · C.I. Solvent Violet (13, 33, 45, 46)
- · C.I. Disperse Violet (22, 24, 26, 28, 31)
- · C.I. Acid Violet (49)
- · C.I. Basic Violet (2, 7, 10)
- · C.I. Solvent Orange (1, 2, 5, 6, 37, 45, 62, 99)
- · C.I. Acid Orange (1, 7, 8, 10, 20, 24, 28, 33, 56, 74)
- · C.I. Direct Orange (1)
- · C.I. Disperse Orange (5)
- · C.I. Direct Brown (6, 58, 95, 101, 173)
- · C.I. Acid Brown (14)

One kind of the component (C) may be used alone, or two or more kinds thereof may be appropriately in combination.

The content of the component (C) in the dental porcelain paste is not particularly limited, and for example, is preferably 0.001 part by mass or more, more preferably 0.002 part by mass or more, further preferably 0.004 part by mass or more, and still further preferably 0.010 part by mass or more, with respect to 100 parts by mass in total of the component (A) and the component (B), from the standpoint of easily allowing the values of $\Delta E^*$, $\Delta L^*$, and $\Delta E1^*$ to be larger values, and for example, is preferably less than 0.60 part by mass, more preferably 0.50 part by mass or less, further preferably 0.40 part by mass or less, and still further preferably 0.30 part by mass or less, with respect to 100 parts by mass in total of the component (A) and the component (B), from the standpoint of suppressing the occurrence of bubbles and carbonization in the resulting baked product.

As described above, the lower limit values and the upper limit values described in a stepwise manner each can be independently combined. For example, in one embodiment of the present invention, the content of the component (C) in the dental porcelain paste is preferably 0.001 part by mass or more and less than 0.60 part by mass, more preferably 0.002 to 0.50 part by mass, further preferably 0.004 to 0.40 part by mass, and still further preferably 0.010 to 0.30 part by mass, with respect to 100 parts by mass in total of the component (A) and the component (B).

The total content of the component (A), the component (B), and the component (C) in the dental porcelain paste is preferably 90% by mass or more, more preferably 95% by mass or more, further preferably 98% by mass or more, still further preferably 99% by mass or more, and still more further preferably 99.5% by mass or more, and is 100% by mass or less, based on the dental porcelain paste as 100% by mass, from the standpoint of facilitating the effects of the present invention.

As described above, the lower limit values and the upper limit values described in a stepwise manner each can be independently combined. For example, in one embodiment of the present invention, the total content of the component (A), the component (B), and the component (C) in the dental porcelain paste is preferably 90 to 100% by mass, more preferably 95 to 100% by mass, further preferably 98 to 100% by mass, and still further preferably 99.5 to 100% by mass, based on the dental porcelain paste as 100% by mass.

(Fluorescent Agent)

The porcelain paste preferably further contains a fluorescent agent from the standpoint of reproducing the fluorescence of the natural tooth.

The kind of the fluorescent agent is not limited, as long as exerting the effects of the present invention, and examples thereof include $Y_2SiO_5$:Ce, $Y_2SiO_5$:Tb, (Y,Gd,Eu)$BO_3$, $Y_2O_3$:Eu, YAG:Ce, $ZnGa_2O_4$:Zn, and $BaMgAl_{10}O_{17}$:Eu.

One kind of the fluorescent agent may be used alone, or two or more kinds thereof may be appropriately used in combination.

In the case where the porcelain paste contains the fluorescent agent, the content of the fluorescent agent is not particularly limited, as far as exerting the effects of the present invention, and is preferably 0.001 part by mass or more, more preferably 0.010 part by mass or more, further preferably 0.050 part by mass or more, and still further preferably 0.10 part by mass or more, and is preferably 0.50 part by mass or less, more preferably 0.45 part by mass or less, further preferably 0.40 part by mass or less, and still further preferably 0.35 part by mass or less, with respect to 100 parts by mass in total of the component (A) and the component (B), from the standpoint of easily reproducing the fluorescence of the natural tooth.

As described above, the lower limit values and the upper limit values described in a stepwise manner each can be independently combined. For example, in one embodiment of the present invention, the content of the fluorescent agent in the dental porcelain paste is preferably 0.001 to 0.50 part by mass, more preferably 0.010 to 0.45 part by mass, further preferably 0.050 to 0.40 part by mass, and still further preferably 0.10 to 0.35 part by mass, with respect to 100 parts by mass in total of the component (A) and the component (B).

(Inorganic Pigment)

The porcelain paste may further contain an inorganic pigment.

The kind of the inorganic pigment is not limited, as long as exerting the effects of the present invention, and examples thereof include praseodymium oxide, vanadium oxide, iron oxide, nickel oxide, chromium oxide, manganese oxide, cerium oxide, a tin oxide compound (for example, a composite oxide containing tin(II) oxide or tin(IV) oxide as a component (such as vanadium-tin yellow and chromium-tin pink)), bismuth-vanadium yellow, vanadium-zirconium yellow, praseodymium yellow, cobalt blue, manganese pink, chromium-alumina pink, chromium iron zinc, titanium oxide ($TiO_2$), zirconium oxide ($ZrO_2$), and zirconium silicate ($ZrSiO_4$).

One kind of the inorganic pigment may be used alone, or two or more kinds thereof may be appropriately used in combination.

In the case where the porcelain paste contains the inorganic pigment, the content of the inorganic pigment is not particularly limited, as long as exerting the effects of the present invention, and for example, is preferably 0.001 part by mass or more, more preferably 0.010 part by mass or more, further preferably 0.050 part by mass or more, and still further preferably 0.10 part by mass or more, and is preferably 0.50 part by mass or less, more preferably 0.45 part by mass or less, further preferably 0.40 part by mass or less, and still further preferably 0.30 part by mass or less, with respect to 100 parts by mass in total of the component (A) and the component (B).

As described above, the lower limit values and the upper limit values described in a stepwise manner each can be independently combined. For example, in one embodiment of the present invention, the content of the inorganic pigment in the dental porcelain paste is preferably 0.001 to 0.50 part by mass, more preferably 0.010 to 0.45 part by mass, further preferably 0.050 to 0.04 part by mass, and still further preferably 0.10 to 0.30 part by mass, with respect to 100 parts by mass in total of the component (A) and the component (B).

<Additional Components>

The porcelain paste may further contain additional components depending on necessity, in addition to the components (A) to (C) and the fluorescent agent and the inorganic pigment, which may be optionally contained, unless the effects of the present invention are impaired. Examples of the additional components include water, a coloring agent other than the component (C), an opacifier, a pH modifier, a polymerization accelerator, and a polymerization initiator.

For example, the dental porcelain paste as one embodiment of the present invention may practically contain water mixed therein in the use thereof. However, water that is practically contained in the dental porcelain material in the use thereof is not considered as a component of the dental porcelain paste as one embodiment of the present invention, as long as satisfying the composition of the dental porcelain paste and exerting the effects of the present invention. In other words, water that is unintentionally mixed in the porcelain paste in the use thereof is different from water as the additional component above.

Examples of the method for producing the dental porcelain paste as one embodiment of the present invention include a production method including at least a step of mixing the component (A), the component (B), the component (C), and the fluorescent agent, the inorganic pigment, and one or more kind selected from the additional components, which may be added depending on necessity. The condition for mixing is not particularly limited, and the components to be mixed may be added at once, or may be added in portions. The kneader used for mixing may be an ordinary kneader. Examples thereof include a mortar, a twin-screw kneader (Twinmix), a three-screw kneader (Trimix), a kneader, and a planetary mixer. Among these, a mortar and a planetary mixer are preferably used.

[Applications and the Likes of Dental Porcelain Paste]

The porcelain paste can be used, for example, for producing a dental prosthesis formed of ceramics, such as an inlay, an onlay, a laminate veneer, and a crown.

The purpose of the porcelain paste is not particularly limited, and can be used, for example, as a body porcelain material (dentin color porcelain material), a cervical porcelain material, an incisal porcelain material (enamel color porcelain material), a translucent porcelain material, an opaque porcelain material, a stain porcelain material, and a glaze porcelain material.

The dental porcelain paste as one embodiment of the present invention before baking has a large color tone difference from the material used in the dental porcelain material forming the substrate, and therefore the coated portion of the porcelain paste coated on the substrate and the uncoated portion thereof can be visually distinguished easily from each other.

In the dental porcelain paste as one embodiment of the present invention, furthermore, the coloration due to the component (C) is decolored after baking, resulting in substantially no color difference from the substrate, and therefore there is substantially no influence on the color tone of the substrate, and also the effect of preventing the occurrence of bubbles and black spots is obtained.

Accordingly, the dental porcelain paste as one embodiment of the present invention is suitable for the application requiring higher distinguishability in the coating operation before baking.

Consequently, as described above, the porcelain paste is preferably used as a stain porcelain material or a glaze porcelain material used for finishing dental prostheses, and more preferably used as a glaze porcelain material.

For example, in the case where the porcelain paste is used as a glaze porcelain material, the porcelain paste is preferably used for finishing a dental prosthesis formed of a ceramic material as described above. Further, known representative examples of the dental prosthesis formed of a ceramic material include dental prostheses of a PFM type, a PFZ type, an FCZ type, and an LDS type as described above.

In the FCZ type among these, a glazing effect through self-glazing cannot be obtained since the surface thereof is formed of zirconia, and therefore the glazing thereof requires a porcelain paste that is necessarily coated to a certain thickness (for example, 20 $\mu$m or more). The porcelain paste is excellent in distinguishability and can be easily controlled in coating thickness as described above, and therefore the porcelain paste can be favorably applied to the FCZ type dental prosthesis among the aforementioned types of dental prostheses.

Examples of other practical embodiments of the present invention include the use of the dental porcelain paste for the treatment of teeth (for example, aesthetic dental treatment, missing tooth treatment, prosthetic dental treatment, such as a prosthetic tooth, and dental caries treatment).

One kind of the porcelain paste may be used alone, or two or more kinds thereof may be appropriately used in combination, as long as exerting the effects of the present invention.

[Dental Porcelain Kit]

The dental porcelain kit as one embodiment of the present invention includes the dental porcelain paste as one embodiment of the present invention described above, and a color reference for being the reference for the coating thickness of the dental porcelain paste.

The dental porcelain paste contained in the dental porcelain kit is the same as described in the section of the dental porcelain paste, and the preferred embodiments thereof are also the same.

The color reference for being the reference for the coating thickness of the dental porcelain paste is used for determining the coating thickness of the porcelain paste based on the color tone of the coating film formed by coating the dental porcelain paste before baking on the particular substrate.

Examples of an embodiment of the color reference are not particularly limited, as long as the color tone in the case where the porcelain paste is coated on the particular substrate, for example, a zirconia base material, to the particular thickness can be visually confirmed by the user. For example, it is possible that the color tone in the case where the porcelain paste is coated on the particular substrate to the particular thickness is reproduced and colored on paper, a plastic sheet, a metal sheet, a ceramic sheet, or the like, which can be visually confirmed by the user of the porcelain paste, and it is also possible to use a photograph or an actual sample of the porcelain paste coated to various thicknesses.

For example, in one embodiment of the color reference for the purpose in which the porcelain paste is coated on the substrate to a thickness in a range of 20 to 40 $\mu$m, the user is enabled to confirm visually the color tones of the porcelain paste that is coated to thicknesses with a certain interval within the range (for example, the color tone at a thickness of 20 $\mu$m and the color tone at a thickness of 25 $\mu$m) or that is within every certain thickness range (for example, the color tone in a thickness of 20 to 25 $\mu$m and the color tone in a thickness of 25 to 30 $\mu$m).

The substrate used for producing the color reference is also not particularly limited. For example, in the case where a base material formed of zirconia is used as the substrate, the color reference may be produced in such a manner that enables to confirm the color tone of the surface of the coating film formed by coating the porcelain paste to the particular thickness on the zirconia base material.

[Method for producing Dental Prosthesis]

The method for producing a dental prosthesis as one embodiment of the present invention includes a step (I) of coating the dental porcelain paste as one embodiment of the present invention on a base material, and a step (II) of baking the dental porcelain paste coated on the base material.

<Step (I)>

In the step (I), the dental porcelain paste is coated on a base material.

The dental porcelain paste used in the step (I) is the same as described in the section of the dental porcelain paste, and the preferred embodiments thereof are also the same.

The "base material" (substrate) that is a target on which the porcelain paste is to be coated in the step (I) is not particularly limited, and may be appropriately selected depending on the purpose of the porcelain paste.

For example, in the case where the porcelain paste is used as a glaze porcelain material, the porcelain paste is preferably used for finishing a dental prosthesis of a ceramic material described above, and examples of the base material of the dental prosthesis of a ceramic material include zirconium oxide (zirconia), aluminum oxide (alumina), feldspar glass, and disilicate glass.

As described above, zirconia among these cannot provide the glazing effect through self-glazing, and therefore the glazing thereof requires the porcelain paste that is necessarily coated to a certain thickness (for example, 20 $\mu$m or more). As described above, the porcelain paste is excellent in distinguishability, and the coating thickness thereof can be easily adjusted. Therefore, the porcelain paste can be favorably applied to the base material formed of zirconia.

The shape of the base material is also not particularly limited, and in the case where the porcelain paste is used as a glaze porcelain material, the porcelain paste is preferably used for finishing a dental prosthesis of a ceramic material described above, and therefore the shape of the base material is preferably processed into the shape of the dental prosthesis.

Examples of the method of coating the porcelain paste on the base material in the step (I) include a method using a writing brush or a painting brush, and spraying may also be employed depending on the kind of the porcelain paste. Furthermore, the base material may be immersed in a container filled with the porcelain paste, and in this case, the inner surface of the base material is preferably sealed in advance for preventing the coating liquid from flowing therein.

The thickness of the coating film formed by coating the porcelain paste may be appropriately set depending on purpose, and for example, in the case where the porcelain paste is used as a glaze porcelain material, is preferably 10 $\mu$m or more, more preferably 15 $\mu$m or more, and further preferably 20 $\mu$m or more, since the glazing can be easily performed with the resulting baked product that has a certain thickness. The thickness thereof is preferably 50 $\mu$m or less, more preferably 45 $\mu$m or less, and further preferably 40 $\mu$m or less, from the standpoint of preventing the entrainment of bubbles due to a too large thickness of the porcelain paste, and the standpoint of reducing the influence of the thickness of the resulting baked product on the occlusion surfaces, the occlusion diameter, and the contacts of the prostheses.

In the step (I), it is preferred to use the color reference for being the reference for the coating thickness of the dental porcelain paste described in the section of the dental porcelain kit. Accordingly, in the step (I), it is preferred to use the dental porcelain kit as one embodiment of the present invention.

As described above, the use of the color reference for being the reference for the coating thickness facilitates the adjustment of the coating thickness to the prescribed thickness even in the case where the coating operation is performed visually and manually.

The color reference for being the reference for the coating thickness of the dental porcelain paste that is preferably used in the step (I) is the same as described in the section of the dental porcelain kit, and the preferred embodiments thereof are also the same.

<Step (II)>

In the step (II), the dental porcelain paste coated on the base material is baked.
The baking temperature (baking maximum temperature) in baking the porcelain paste can be appropriately set depending on the kind and the use mode of the porcelain paste, the kind of the substrate, and the like, and is not particularly limited, as long as being a temperature at which the component (C) is discolored through baking, and the baking temperature is preferably 700°C or more, more preferably 720°C or more, and further preferably 730°C or more, and is preferably 1,050°C or less, more preferably 1,000°C or less, and further preferably 980°C or less.
As described above, the lower limit values and the upper limit values described in a stepwise manner each can be independently combined. For example, in one embodiment of the present invention, the baking temperature in the step (II) is preferably 700 to 1,050°C, more preferably 720 to 1,000°C, and further preferably 730 to 980°C.
The temperature rise rate in baking to the maximum baking temperature may be appropriately changed depending on the kind of the porcelain material, and is not particularly limited, and is preferably 10 to 70°C/min, more preferably 15 to 60°C/min, and further preferably 20 to 50°C/min.
Before baking, the porcelain paste may be dried, and the drying condition is not particularly limited. The baking may be vacuum baking preformed in vacuum since the bubbles existing inside the glass powder (A) can be largely reduced to provide a dental prosthesis excellent in transparency, resulting in excellent aesthetic quality. The degree of vacuum in the vacuum baking is not particularly limited, and may be, for example, 750 mmHg or less, and may be 730 mmHg or less. The temperature at the start of the vacuum baking is 3not particularly limited, and may be, for example, 550 to 700°C. In addition, the vacuum baking may be performed by heating under vacuum condition in raising the temperature, releasing to the atmospheric pressure in reaching the baking maximum temperature, and retaining under the atmospheric condition.
The present invention encompasses embodiments including various combinations of the aforementioned configurations within the scope of the technical concept of the present invention, as long as exerting the effects of the present invention.

Examples

The present invention will be described more specifically with reference to examples below, but the present invention is not limited to the examples.

<Production of Glass Powder>

Amorphous type potassium aluminosilicate glass frit formed of $SiO_2$, $Al_2O_3$, ZnO, $K_2O$, $Na_2O$, $Li_2O$, CaO, MgO, $Sb_2O_3$, $CeO_2$, and BaO was pulverized with a planetary mill, so as to produce powder having an average particle diameter shown in Tables 1 and 2 below. For the average particle diameter, the volume based average particle diameter (D50) was measured by the laser diffraction scattering method. The measuring apparatus used was " Microtrac (registered trade name) MT3300II " (available from MicrotracBEL Corporation), and water was used as the dispersion medium.

[Examples 1 to 14 and Comparative Example 1]

The components shown in Tables 1 and 2 were mixed at the contents (part by mass) shown in Tables 1 and 2 with a mortar under ordinary temperature (25°C) for approximately 10 minutes, so as to prepare dental porcelain pastes

[Evaluation of Dental Porcelain Paste]

The porcelain pastes prepared in Examples and Comparative Example each were measured for the properties in the following manner. The resulting evaluation results are shown in Tables 1 and 2 below.

<Evaluation of Color Tone of Dental Porcelain Paste before Baking>

The porcelain pastes each were placed inside a stainless steel ring (inner diameter: 15 mm, thickness: 0.5 mm), which was held with two plies of slide glass ("AZ Labo (registered trade name) Slide Glass", available from AZ One Corporation, width: 25 mm, length: 75 mm, thickness: 1 mm) from the upper and lower sides under pressure, and the L*a*b* values (JIS Z8781-4:2013, Colorimetry Part 4: CIE 1976 L*a*b* color space) of white background were measured with a spectral colorimeter ("CM-3610A", available from Konica Minolta Inc., D65 light source, geometric condition c (di: 8°, de: 8°), diffused illumination: 8° light reception, easurement mode: SCI, measurement diameter/illumination diameter = $\phi$ mm/$\phi$11 mm). The result obtained was designated as the color tone L1*, a1*, b1* of the dental porcelain paste before baking (n = 3).

<Evaluation of Color Tone of Baked Product after baking Porcelain Paste>

The porcelain pastes each were baked in vacuum at a maximum baking temperature of 740°C, and polished with #1500 polishing paper to provide a baked product having an inner diameter of 15 mm and a thickness of 0.5 mm. The other conditions than the maximum baking temperature were a drying time before baking of 6 minutes, a baking start temperature of 400°C, a temperature rise rate of 45°C/min, a vacuum start temperature of 650°C, a vacuum degree of 720 mmHg, and releasing to the atmospheric pressure (vacuum release) at the time reaching 740°C, followed by retaining for 1 minute, then quenching to room temperature (25°C).

The resulting baked product was held with two plies of slide glass ("AZ Labo (registered trade name) Slide Glass", available from AZ One Corporation, width: 25 mm, length: 75 mm, thickness: 1 mm) from the upper and lower sides under pressure, and the L*a*b* values (JIS Z8781-4:2013, Colorimetry Part 4: CIE 1976 L*a*b* color space) of white background were measured with a spectral colorimeter ("CM-3610A", available from Konica Minolta Inc., D65 light source, geometric condition c (di: 8°, de: 8°), diffused illumination: 8° light reception, measurement mode: SCI, measurement diameter/-illumination diameter = $\phi$8 mm/$\phi$11 mm). The result obtained was designated as the color tone L2*, a2*, b2* of the baked product (n = 3).

<Calculation of Color Tone Difference before and after Baking>

The change in color tone (color tone difference) of the porcelain paste before and after baking was calculated from the color tone of the porcelain paste before baking and the color tone of the baked product obtained by baking the porcelain paste according to the following calculation expression.

$$\Delta E^* = \{(L2^*\text{-}L1^*)^2+(a2^*\text{-}a1^*)^2+(b2^*\text{-}b1^*)^2\}^{1/2}$$

$$\Delta L^* = |L2^*\text{-}L1^*|$$

$\Delta E^*$ above is the color difference $\Delta E^*$ relating to the condition (1), and $\Delta L^*$ above is the brightness $\Delta L^*$ relating to the condition (2).

<Production of Base Material>

A base material for the evaluation described later was produced.
A specimen having a disk shape having a diameter of 19 mm and a thickness of 1.6 mm was machined from zirconia for dental use ("KATANA (registered trade name) Zirconia STML A3", thickness: 14 mm, available from Kuraray Noritake Dental, Inc.) with a dental milling machine DWX-51D (available from Roland DG Corporation), resulting in a shaped article.
With a baking furnace available from SK Medical Electronics Co., Ltd. (trade name: "Noritake Katana (registered trade name) F-1N"), the shaped article was started to bake from room temperature (25°C) in the atmosphere, heated at a temperature rise rate of 10°C/min to 1,550°C, and baked at 1,550°C for 2 hours (atmospheric baking), and then the baked product was polished with #1500 polishing paper to provide a base material formed of a zirconia sintered article having a disk shape having a diameter of 14 mm and a thickness of 1.2 mm.

<Evaluation of Distinguishability of Porcelain Paste>

The porcelain pastes each were coated on the base material produced above, and the distinguishability of the coated portion was evaluated by the following criteria (n = 3).
A: The portion having the paste coated thereon was clearly distinguished visually.
F: The color tone difference between the base material and the porcelain paste was unclear, and it was difficult to distinguish the coated portion visually.

<Calculation of Color Difference ΔE1* between Porcelain Paste before Baking and Base Material>

The base material produced above was measured for the L*a*b* values (JIS Z8781-4:2013, Colorimetry Part 4: CIE 1976 L*a*b* color space) of white background with a spectral colorimeter ("CM-3610A", available from Konica Minolta Inc., D65 light source, geometric condition c (di: 8°, de: 8°), diffused illumination: 8° light reception, measurement mode: SCI, measurement diameter/illumination diameter = $\phi$8 mm/$\phi$11 mm). The result obtained was designated as the color tone L3*, a3*, b3* of the base material (n = 3). The color tone of the base material was L3* = 73.4, a3* = 4.0, b3* = 27.4.

Subsequently, a stainless steel ring (inner diameter: 15 mm, thickness: 0.5 mm) was placed on the base material, and the porcelain pastes prepared each were placed in the ring. Thereafter, one ply of slide glass ("AZ Labo (registered trade name) Slide Glass", available from AZ One Corporation, width: 25 mm, length: 75 mm, thickness: 1 mm) was placed from the above under pressure, then the porcelain paste was colorimetrically measured (L*a*b* color system) with a spectral colorimeter ("CM-3610A", available from Konica Minolta Inc.) under the same condition as above, and the result obtained was designated as the color tone L4*, a4*, b4* of the paste after coating (n = 3).

The color difference ΔE1* between the dental porcelain paste before baking and the base material was calculated according to the following calculation expression.

$$\Delta E1^* = \{(L4^*\text{-}L3^*)^2 + (a4^*\text{-}a3^*)^2 + (b4^*\text{-}b3^*)^2\}^{1/2}$$

<Evaluation of Bubbles and Carbonization after Baking>

The porcelain pastes prepared each were coated on the base material produced above, and baked in vacuum at 740°C to provide a baked product. The baked product was observed with an optical microscope ("Digital Microscope KH-7700", available from Hirox Co., Ltd.) at a magnification of 100, and evaluated for the presence of bubbles and carbonization.

As the evaluation criteria, an ordinary dental porcelain material ("Cerabian (registered trade name) ZR FC Paste Stain Clear Glaze", available from Kuraray Noritake Dental, Inc.) was baked in vacuum at 750°C to provide a baked product, which was observed with the same optical microscope in the same manner, and the result was compared thereto and evaluated as follows (n = 3).

A: Bubbles equivalent to the ordinary dental porcelain material were observed, and no black spot was observed.

B: More bubbles than the ordinary dental porcelain material were observed but were not practically problematic, and no black spot was observed.

F: Distinctly more bubbles than the ordinary dental porcelain material were observed, or black sports were observed, or the both problems occurred.

<Calculation of Color Difference ΔE2* between Baked Product and Base Material>

The base material produced above was measured for the L*a*b* values (JIS Z8781-4:2013, Colorimetry Part 4: CIE 1976 L*a*b* color space) of white background with a spectral colorimeter ("CM-3610A", available from Konica Minolta Inc., D65 light source, geometric condition c (di: 8°, de: 8°), diffused illumination: 8° light reception, measurement mode: SCI, measurement diameter/illumination diameter = $\phi$8 mm/$\phi$11 mm). The result obtained was designated as the color tone L3*, a3*, b3* of the base material (n = 3). The color tone of the base material was L3* = 73.4, a3* = 4.0, b3* = 27.4.

Subsequently, the porcelain pastes prepared each were coated on the base material produced to make a thickness of the baked product after baking of 65 $\mu$m, and then baked in vacuum at a maximum baking temperature of 740°C to provide a baked product. The other conditions than the maximum baking temperature were a drying time before baking of 6 minutes, a baking start temperature of 400°C, a temperature rise rate of 45°C/min, a vacuum start temperature of 650°C, a vacuum degree of 720 mmHg, and releasing to the atmospheric pressure (vacuum release) at the time reaching 740°C, followed by retaining for 1 minute, then quenching to room temperature (25°C).

Each of the resulting baked product was colorimetrically measured (L*a*b* color system) with a spectral colorimeter ("CM-3610A", available from Konica Minolta Inc.) under the same condition as above, and the result obtained was designated as the color tone L5*, a5 *, b5* of the baked product. The color difference ΔE2* between the baked product obtained by baking the porcelain paste and the base material was calculated according to the following calculation expression (n = 3).

$$\Delta E2^* = \{(L5^*\text{-}L3^*)^2 + (a5^*\text{-}a3^*)^2 + (b5^*\text{-}b3^*)^2\}^{1/2}$$

Table 1

| Component | Compound | Example 1 | Example 2 | Example 3 | Example 4 | Example 5 | Example 6 | Example 7 | Example 8 |
|---|---|---|---|---|---|---|---|---|---|
| Glass powder (A) | Average particle diameter: 5.0 μm | 60.6 | 60.6 | 60.6 | 60.6 | 60.6 | 60.6 | 60.6 | 64.3 |
| | Average particle diameter: 25.0 μm | | | | | | | | |
| Organic solvent (B) | Isoprene glycol | 39.4 | | 39.4 | 39.4 | 39.4 | 39.4 | 39.4 | 35.7 |
| | 2-Phenoxyethanol | | 39.4 | | | | | | |
| Coloring agent (C) | Solvent Blue 44 | 0.024 | 0.024 | 0.012 | 0.006 | 0.003 | 0.0015 | | 0.024 |
| | Solvent Red 27 | | | | | | | 0.024 | |
| Fluorescent agent | $Y_2SiO_5$:Ce | 0.3 | 0.3 | 0.3 | 0.3 | 0.3 | 0.3 | 0.3 | 0.3 |
| Pigment | Zircon based ($ZrSiO_4$ based) pigment | | | | | | | | |
| Total | | 100.3 | 100.3 | 100.3 | 100.3 | 100.3 | 100.3 | 100.3 | 100.3 |
| Color tone before baking (white background) (*1) | L1* | 48.0 | 46.0 | 56.3 | 62.8 | 63.6 | 66.9 | 52.4 | 51.2 |
| | a1* | -22.7 | -32.4 | -28.9 | -28.2 | -18.6 | -8.2 | 36.4 | -22.1 |
| | b1* | -33.1 | -20.7 | -31.5 | -23.6 | -13.0 | -3.8 | 10.1 | -31.1 |
| Color tone after baking (white background) (*2) | L2* | 82.7 | 82.7 | 82.7 | 82.7 | 82.7 | 82.7 | 82.7 | 82.7 |
| | a2* | -0.4 | -0.4 | -0.4 | -0.4 | -0.4 | -0.4 | -0.4 | -0.4 |
| | b2* | 2.8 | 2.8 | 2.8 | 2.8 | 2.8 | 2.8 | 2.8 | 2.8 |
| Color tone difference before and after baking (white background) | ΔE* | 54.7 | 54.0 | 51.8 | 43.2 | 30.8 | 18.8 | 48.2 | 51.0 |
| | ΔL* | 34.7 | 36.6 | 26.3 | 19.8 | 19.1 | 15.8 | 30.2 | 31.4 |
| Before baking | Distinguishability of porcelain paste | A | A | A | A | A | A | A | A |
| | Color tone difference ΔE1* between porcelain paste (thickness: 0.5 mm) and base material | 53.3 | 52.1 | 47.5 | 42.0 | 33.2 | 16.2 | 40.7 | 49.2 |

(continued)

| Component | Compound | Example 1 | Example 2 | Example 3 | Example 4 | Example 5 | Example 6 | Example 7 | Example 8 |
|---|---|---|---|---|---|---|---|---|---|
| After baking | Bubbles and carbonization | A | A | A | A | A | A | A | A |
| | Color tone difference ΔE2* between baked product (thickness: 65 μm) and base material | 1.1 | 1.2 | 1.0 | 0.9 | 1.0 | 1.1 | 1.1 | 1.2 |

(*1) Color tone of porcelain paste before baking (thickness: 0.5 mm)
(*2) Color tone of baked product (thickness: 0.5 mm) obtained by baking porcelain paste

Table 2

| Component | Compound | Example 9 | Example 10 | Example 11 | Example 12 | Example 13 | Example 14 | Comparative Example 1 |
|---|---|---|---|---|---|---|---|---|
| Glass powder (A) | Average particle diameter: 5.0 μm | | 60.6 | 60.6 | 60.6 | 60.6 | 60.6 | 60.6 |
| | Average particle diameter: 25.0 μm | 60.6 | | | | | | |
| Organic solvent (B) | Isoprene glycol | 39.4 | 39.4 | 39.4 | 39.4 | 39.4 | 39.4 | |
| | 2-Phenoxyethanol | | | | | | | 39.4 |
| Coloring agent (C) | Solvent Blue 44 | 0.024 | 0.024 | 0.024 | 0.10 | 0.25 | 0.50 | |
| | Solvent Red 27 | | | | | | | |
| Fluorescent agent | $Y_2SiO_5$:Ce | 0.3 | | | 0.3 | 0.3 | 0.3 | 0.3 |
| Pigment | Zircon based ($ZrSiO_4$ based) pigment | | | 0.2 | | | | |
| Total | | 100.3 | 100.0 | 100.2 | 100.4 | 100.6 | 100.8 | 100.3 |
| Color tone before baking (white background) (*1) | L1* | 46.0 | 48.1 | 48.3 | 38.8 | 31.9 | 28.3 | 76.2 |
| | a1* | -22.8 | -22.7 | -22.9 | -13.9 | -5.3 | -1.9 | 0.8 |
| | b1* | -32.1 | -33.2 | -33.3 | -38.7 | -35.5 | -26.7 | 8.1 |
| Color tone after baking (white background) (*2) | L2* | 82.3 | 82.7 | 82.8 | 82.4 | 82.3 | 81.8 | 82.6 |
| | a2* | -0.4 | -0.4 | -0.4 | -0.9 | -0.9 | -0.9 | -0.4 |
| | b2* | 2.7 | 2.7 | 2.7 | 2.7 | 2.6 | 2.5 | 2.9 |
| Color tone difference before and after baking (white background) | ΔE* | 55.1 | 54.6 | 54.8 | 61.5 | 63.3 | 61.0 | 8.4 |
| | ΔL* | 36.4 | 34.6 | 34.6 | 43.6 | 50.4 | 53.5 | 6.4 |
| Before baking | Distinguishability of porcelain paste | A | A | A | A | A | A | F |
| | Color tone difference ΔE1* between porcelain paste (thickness: 0.5 mm) and base material | 53.1 | 53.2 | 54.0 | 76.7 | 75.9 | 70.7 | 10.6 |

(continued)

| Component | Compound | Example 9 | Example 10 | Example 11 | Example 12 | Example 13 | Example 14 | Comparative Example 1 |
|---|---|---|---|---|---|---|---|---|
| After baking | Bubbles and carbonization | A | A | A | A | A | B | A |
| | Color tone difference $\Delta E2^*$ between baked product (thickness: 65 $\mu$m) and base material | 1.1 | 0.9 | 0.9 | 1.3 | 1.6 | 2.2 | 1.3 |

(*1) Color tone of porcelain paste before baking (thickness: 0.5 mm)
(*2) Color tone of baked product (thickness: 0.5 mm) obtained by baking porcelain paste

It is understood from the results in Tables 1 and 2 that the porcelain pastes of Examples 1 to 14 containing the components (A) to (C) and satisfying at least one condition selected from the condition (1) and the condition (2) have a large color difference $\Delta E1^*$ between the porcelain paste before baking and the base material having the paste coated thereon, and thus are excellent in distinguishability in the coating operation before baking.

It is also understood that the porcelain pastes of Examples 1 to 14 have a reduced color difference $\Delta E2^*$ between the baked product obtained by baking the porcelain paste and the base material, and thus there is substantially no influence on the color tone of the base material as the substrate. Furthermore, the porcelain pastes of Examples 1 to 13 cause bubbles in the baked product equivalent to the ordinary dental porcelain material, and have no black spot occurring therein. The porcelain paste of Example 14 causes more bubbles in the baked product than the ordinary dental porcelain material, which however causes no practical problem, and has no black spot occurring therein.

Accordingly, it is understood that the dental porcelain paste as one embodiment of the present invention is suitable for the application requiring higher distinguishability in the coating operation before baking.

Industrial Applicability

As described above, the dental porcelain paste as one embodiment of the present invention before baking has a large color tone difference from the base material as the substrate, and therefore the coated portion of the porcelain paste coated on the substrate and the uncoated portion thereof can be visually distinguished easily from each other. Accordingly, the dental porcelain paste as one embodiment of the present invention can avoid the occurrence of uncoated areas of the porcelain paste and can facilitate the adjustment of the coating thickness thereof, and therefore is excellent in distinguishability in the coating operation before baking.

Furthermore, the dental porcelain paste as one embodiment of the present invention changes the color tone thereof after baking, resulting in substantially no color difference from the substrate, and therefore there is substantially no influence on the color tone of the substrate, and also the effect of preventing the occurrence of bubbles and black spots is obtained.

Consequently, the dental porcelain paste as one embodiment of the present invention can be favorably used, for example, as a glaze porcelain material.

The situations in recent years including the increasing demand for ceramic prostheses and the increasing individual demand for aesthetic quality are expected to lead toan increased use frequency of dental porcelain materials, and therefore the dental porcelain paste as one embodiment of the present invention is useful therefor.

**Claims**

1. A dental porcelain paste comprising glass powder (A), an organic solvent (B), and a coloring agent (C) decolorable by baking, and satisfying at least one condition selected from the following conditions (1) and (2):

   condition (1): a color difference $\Delta E^*$ calculated from a color tone obtained by colorimetrically measuring in the L*a*b* color space the dental porcelain paste having a thickness of 0.5 mm before baking and a color tone obtained by colorimetrically measuring in the L*a*b* color space a baked product having a thickness of 0.5 mm obtained by baking the dental porcelain paste in vacuum at 740°C is 8.5 or more, and
   condition (2): a brightness difference $\Delta L^*$ calculated from a brightness obtained by colorimetrically measuring in the L*a*b* color space the dental porcelain paste having a thickness of 0.5 mm before baking and a brightness obtained by colorimetrically measuring in the L*a*b* color space a baked product having a thickness of 0.5 mm obtained by baking the dental porcelain paste in vacuum at 740°C is 6.5 or more.

2. The dental porcelain paste according to claim 1, wherein a color difference $\Delta E1^*$ calculated from a color tone obtained by colorimetrically measuring in the L*a*b* color space a base material and a color tone obtained by colorimetrically measuring in the L*a*b* color space the dental porcelain paste coated on the base material to a thickness of 0.5 mm is 11.0 or more.

3. The dental porcelain paste according to claim 1 or 2, wherein a color difference $\Delta E2^*$ calculated from a color tone obtained by colorimetrically measuring in the L*a*b* color space a base material and a color tone obtained by colorimetrically measuring in the L*a*b* color space a baked product obtained by coating the dental porcelain paste on the base material to make a thickness after baking of 65 $\mu$m, and baking in vacuum at 740°C is 3.0 or less.

4. The dental porcelain paste according to any one of claims 1 to 3, wherein the dental porcelain paste has a content of the component (C) of 0.001 part by mass or more and less than 0.60 part by mass with respect to 100 parts by mass in total of the components (A) and (B).

5. The dental porcelain paste according to any one of claims 1 to 4, wherein the component (C) comprises an organic colorant that is dissolvable in the component (B).

6. The dental porcelain paste according to claim 5, wherein the organic colorant is an aromatic based organic colorant.

7. The dental porcelain paste according to claim 5 or 6, wherein the organic colorant is at least one kind selected from the group consisting of an anthraquinone based compound, an azo based compound, a xanthene based compound, a porphyrin based compound, a phthalocyanine based compound, and a triarylmethane base compound.

8. The dental porcelain paste according to any one of claims 1 to 7, which comprises a fluorescent agent.

9. The dental porcelain paste according to claim 8, wherein the dental porcelain paste has a content of the fluorescent agent of 0.001 to 0.50 part by mass with respect to 100 parts by mass in total of the components (A) and (B).

10. The dental porcelain paste according to any one of claims 1 to 9, which comprises an inorganic pigment.

11. A dental porcelain kit comprising the dental porcelain paste according to any one of claims 1 to 10 and a color reference for being the reference for a coating thickness of the dental porcelain paste.

12. A method for producing a dental prosthesis, comprising a step (I) of coating the dental porcelain paste according to any one of claims 1 to 10 on a base material, and a step (II) of baking the dental porcelain paste coated on the base material.

13. The method for producing a dental prosthesis according to claim 12, wherein a baking temperature in the step (II) is 700°C or more.

14. The method for producing a dental prosthesis according to claim 12 or 13, wherein the step (I) includes using a color reference for being the reference for a coating thickness of the dental porcelain paste.

## INTERNATIONAL SEARCH REPORT

| International application No. |
| --- |
| **PCT/JP2023/032152** |

| A. | CLASSIFICATION OF SUBJECT MATTER |
| --- | --- |

*A61K 6/15*(2020.01)i; *A61K 6/20*(2020.01)i; *A61K 6/60*(2020.01)i; *A61K 6/77*(2020.01)i; *A61K 6/836*(2020.01)i
FI:    A61K6/15; A61K6/77; A61K6/60; A61K6/20; A61K6/836

According to International Patent Classification (IPC) or to both national classification and IPC

| B. | FIELDS SEARCHED |
| --- | --- |

Minimum documentation searched (classification system followed by classification symbols)

A61K6/15; A61K6/20; A61K6/60; A61K6/77; A61K6/836

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | WO 2022/118905 A1 (KURARAY NORITAKE DENTAL INC.) 09 June 2022 (2022-06-09) claims, paragraphs [0001]-[0003], [0024]-[0026], [0046]-[0048], examples | 1-14 |
| Y | KR 10-2014-0056832 A (GENOSS CO., LTD.) 12 May 2014 (2014-05-12) claims, paragraphs [0005]-[0017], examples | 1-14 |
| A | JP 2017-193492 A (KURARAY NORITAKE DENTAL INC.) 26 October 2017 (2017-10-26) claims, examples | 1-14 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 November 2023** | **21 November 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2023/032152**

| Patent document cited in search report | Publication date (day/month/year) | Patent family member(s) | Publication date (day/month/year) |
|---|---|---|---|
| WO 2022/118905 A1 | 09 June 2022 | (Family: none) | |
| KR 10-2014-0056832 A | 12 May 2014 | (Family: none) | |
| JP 2017-193492 A | 26 October 2017 | (Family: none) | |

Form PCT/ISA/210 (patent family annex) (January 2015)

**EP 4 582 073 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017193492 A
- JP 2009207743 A